# EUROPEAN PATENT APPLICATION

(11) **EP 3 301 091 A1**
(43) Date of publication of application: **04.04.2018**
(21) Application number: 16191553.3
(22) Date of filing: 29.09.2016
(51) Int. Cl.: C07D 307/68, C08G 63/181, C08G 63/78, C08G 63/90, C08G 63/91

(54) **A PROCESS TO PREPARE A CYCLIC OLIGOMER AND A CYCLIC OLIGOMER OBTAINABLE THEREBY AND A PROCESS TO POLYMERIZE IT**

(71) Applicant: Sulzer Chemtech AG, 8404 Winterthur (CH)
(72) Inventor: The designation of the inventor has not yet been filed
(74) Representative: Henkel, Breuer & Partner

(57) **Abstract**

A process to prepare a cyclic polyester oligomer composition comprising a cyclic polyester oligomer having furanic units is disclosed. The process comprises the step of reacting a monomer component in a ring closing oligomerization step carried out in the presence of a solvent. Linear oligomeric polyester species are separated and removed by one or more sub-steps of adding a zeolite and absorbing impurities onto the zeolite, separating the zeolite having absorbed impurities, cooling the cyclic oligomeric composition and/or adding an anti-solvent in order to precipitate out the cyclic polyester oligomers. The invention further relates to a cyclic polyester oligomer composition obtainable by said process and also to its use in the production of a polyester polymer, particularly by polymerization in the presence of a plasticizer, as well as the polyester polymer obtained thereby. [Fig. 1]

## Description

### BACKGROUND OF THE INVENTION

The present invention relates to a process to prepare a cyclic polyester oligomer composition comprising a cyclic polyester oligomer having furanic units, said oligomer composition obtainable thereby, as well as the use of said cyclic polyester oligomer composition in the production of a polyester polymer, particularly in the presence of a plasticizer, and the polymer obtainable thereby.

Biobased polymers having aromatic building blocks are highly sought today due to their environmentally friendliness and good mechanical properties. An interesting class of biobased aromatic monomers are the furanics such as furan-2,5-dicarboxylic acid (FDA), 5-(hydroxymethyl)furan-2-carboxylic acid (HMFA), and 2,5-bis(hydroxyl methyl)furan (BHMF), which may be prepared from the intermediates furfural (2-furan carboxaldehyde) and 5-hydroxymethyl 2-furan carboxaldehyde (HMF) which may be produced by the acid-catalyzed thermal dehydration of pentoses (C5) and hexoses (C6). The chemical similarity of the furan ring to the phenyl ring makes it possible to replace phenyl-based polymers such as polyethylene terephthalate (PET) by furan-based polymers.

The production of polyesters from furanic building blocks by polycondensation reactions involving heating a mixture of diols and diacids or diesters (monomers) at high temperatures in the presence of organometallic or acid catalyst is known, for example, from US 2,551,731 and US 8,143,355 B2. To allow the progress in this equilibrium reaction towards the formation of the polymer, the formed water or side products such as alcohol must be removed, typically by reduced pressure or gas streams at elevated temperatures in the process. Therefore complex and costly reaction and devolatilization equipments are required. If the polycondensation and devolatilization is insufficient, then high molecular weight polyester having useful mechanical and other properties will not be produced. Furthermore the high temperatures and long residence times lead often to undesired side reactions such as degradation of the monomer, oligomer or polymer, formation of intermolecular bonds leading to branching, and oxidation of the final product with the consequent color development.

Recently new alternative raw materials to the diol and diacid or diester monomers conventionally used to prepare polyesters from furanic building blocks in industrial scale polymerization plants have been developed. WO2014/139603 (A1) discloses that cyclic polyester oligomers may be readily prepared in a process comprising the step of reacting a monomer component in a ring closing oligomerization step under conditions of a reaction temperature and reaction time sufficient to yield a cyclic polyester oligomer having furanic units. Such cyclic polyester oligomers advantageously do not produce large quantities of water or alcoholic side products nor do they require complex reaction and high-capacity devolatilization equipment or harsh high temperature reaction and devolatization steps in order to drive the polymerization to completion as disclosed in WO2014/139602 A1.

Nonetheless low levels of diacid, diol and acidol monomeric and/or dimeric and/or oligomeric species are present at equilibrium in the cyclic polyester oligomer of WO '603, and it was reported that they should be removed so that they would not detrimentally affect the storage stability of the oligomer or its subsequent polymerization processing behavior. It was disclosed that these undesired impurities could be removed from the oligomer product by conventional methods such as chromatography, selective precipitation, distillation, extraction, and crystallization, and a combination of filtration and chromatography was taught by example. Although such purification methods are effective, it is nonetheless desirable to have ones that are as - or preferably more - effective and selective and that do not require large quantities of solvents, long times or hybrid combinations, particularly for large-scale commercial production.

Although the production of polyester polymers by melt polymerization from such cyclic oligomers has been shown to be possible under milder thermal conditions (minimizing color and degradation) and with simpler equipment than polycondensation reactions in WO '602, nonetheless some cyclic oligomeric species have quite high melting points. For example, the often dominant cyclic dimer has a melting point of about 370 °C. Thus although such oligomeric compositions may readily polymerize at milder temperatures below 300 °C, significantly higher temperatures and harsher conditions may be required to melt the raw materials prior to their polymerization thereby causing degradation and discoloration of the thermally sensitive partially aliphatic oligomers.

In conclusion, it would be desirable to have improved processes to both produce such cyclic polyester oligomers as well as to polymerize them.

### SUMMARY OF THE INVENTION

Starting from this state of the art, it is an object of the invention to provide an improved process to prepare a cyclic polyester oligomer having furanic units for use in producing polyester polymers having furanic units and that do not suffer from the previous mentioned deficiencies, particularly. A related object is provide an improved process to use said cyclic polyester oligomer composition in the production of a polyester polymer.

In the present invention, "furanic units" refers to furan derivatives such as those based on the monomers FDA, HMFA, BHMF and their partially or fully reacted mono-ester or diester derivatives. Having furanic units means that the fully or partially reacted derivative of such monomers is incorporated into the cyclic polyester oligomer.

According to the invention, these objects are achieved by a process to prepare a cyclic polyester oligomer composition comprising a cyclic polyester oligomer having furanic units, wherein the process comprises:
- a step of either:
   (I) reacting a monomer component C¹ or D¹ in the presence of an optional catalyst and/or optional organic base in a ring closing oligomerization step under conditions of a reaction temperature and reaction time sufficient to yield a cyclic polyester oligomer having furanic units of structure Y¹, wherein the monomer component C¹ comprises the structure 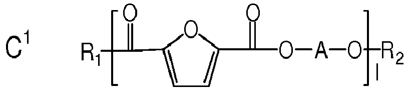 and wherein each of the groups A is an optionally-substituted linear, branched or cyclic alkyl, phenyl, aryl, or alkylaryl, and wherein I is an integer from 1 to 100, preferably 2 to 50, most preferably 3 to 25, and wherein
      R₁= OH, OR, halogen, or O-A-OH,
      R = optionally substituted linear, branched or cyclic alkyl, phenyl, aryl or alkylaryl, 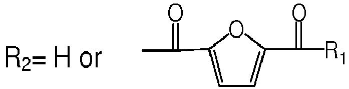
      wherein the monomer component D¹ comprises the structures 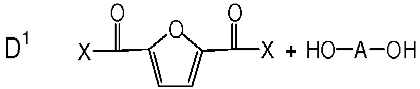
      and wherein A is an optionally-substituted linear, branched or cyclic alkyl, phenyl, aryl, or alkylaryl, and wherein each of the groups X is an OH, a halogen, or an optionally-substituted alkyloxy, phenoxy, or aryloxy, and wherein the groups X are not OH when A is n-butyl, and wherein the structure Y¹ of the cyclic polyester oligomer having furanic units is 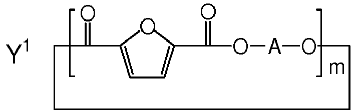 wherein m is an integer from 1 to 20, preferably 2 to 15, most preferably 3 to 10,
      OR
   (II) reacting a monomer component C² or D² in the presence of an optional catalyst and/or optional organic base in a ring closing oligomerization step under conditions of a reaction temperature and reaction time sufficient to yield a cyclic polyester oligomer having furanic units of structure Y²,
      wherein the monomer component C² comprises the structure 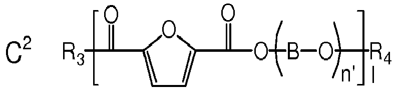 and wherein each of the groups B is an optionally-substituted linear, branched or cyclic alkyl, phenyl, aryl, or alkylaryl, wherein I is an integer as defined above, and wherein n' is an integer from 1 to 20, preferably 2 to 10, and wherein
      R₃= OH, OR, halogen, or O-(B-O)ₙ-H,
      R = optionally substituted linear, branched or cyclic alkyl, phenyl, aryl or alkylaryl, 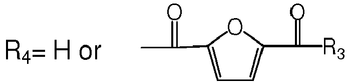
      wherein the monomer component D² comprises the structures 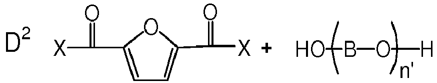 and wherein each of the groups X is an OH, a halogen, or an optionally-substituted alkyloxy, phenoxy, or aryloxy, each of the groups B is an optionally-substituted linear, branched or cyclic alkyl, phenyl, aryl, or alkylaryl, and n' is an integer as defined above,
      and wherein the structure Y² of the cyclic polyester oligomer having furanic units is 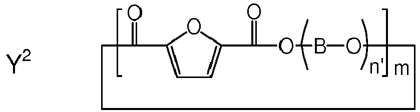 wherein each of the groups B is an optionally-substituted linear, branched or cyclic alkyl, phenyl, aryl, or alkylaryl, n' is an integer as defined above, and m is an integer from 1 to 20, preferably 2 to 15, most preferably 3 to 10,
   AND
- a subsequent step (III) in which linear oligomeric polyester species having furanic units are separated and removed from the cyclic oligomeric composition, wherein
- the reacting of the monomer component C1 or D1 or C2 or D2 in the presence of an optional catalyst and/or optional organic base in a ring closing oligomerization step is carried out in the presence of a solvent, wherein the solvent is selected from the group consisting of an ionic liquid, an optionally-substituted napthalene, optionally-substituted aromatic compound, and their mixtures,
   and optionally in the presence of a zeolite and absorbing impurities onto the zeolite,
- and the subsequent step (III) in which linear oligomeric polyester species having furanic units are separated and removed from the cyclic oligomeric composition comprises one or more of the following sub-steps:
- adding a zeolite and absorbing impurities onto the zeolite,
- cooling the cyclic oligomeric composition in order to precipitate out cyclic polyester oligomers having furanic units,
- adding an antisolvent in order to precipitate out cyclic polyester oligomers having furanic units,
- separating zeolites having absorbed impurities from the cyclic oligomeric composition.

According to the invention, these further objects are achieved firstly by a cyclic polyester oligomer composition obtainable by said process, wherein the composition contains less than 5, preferably 3, most preferably 1 weight % of linear oligomeric polyester species based on the total weight of the composition.

Said cyclic polyester oligomer is used in accordance with the invention in the production of a polyester polymer.

The present invention achieves these objects and provides a solution to this problem by means of a process to prepare a cyclic oligomer composition comprising a cyclic polyester oligomer having furanic units and having either structure Y¹ or Y². These cyclic oligomers advantageously are readily prepared with a high purity in relatively simple and scalable processes in a high conversion and with a low content of linear monomeric or oligomeric species having alcoholic and/or acidic end groups.

These results are first of all surprisingly achieved by carrying out the cyclization reaction in the presence of a solvent. The use of solvent strongly favours the formation cyclic oligomers at much lower conversion levels of end groups in dilute reaction systems versus that of bulk systems. Without wishing to be bound by a particular mechanism, the inventors believe this effect may be due to the presence of the solvent influencing the competition between the two independent reversible reactions of step growth polymerization and cyclization. The higher boiling aromatic solvents have been found to provide good solubility, but preferably the more volatile ones such as xylenes are selected in order to ease their subsequent removal.

The use of zeolites has been found to be particularly effective in removing linear monomeric and oligomeric species versus cyclic species due to the more flexible structure and smaller cross-sectional area of the linear species. In particular, zeolites offer the possibility to tailor their selectivity for linear versus cyclic species and even for various chain lengths. This aspect is particularly beneficial when the achieved purity is greater than 99 %, as then the need for anti-solvents and their subsequent removal is eliminated. Furthermore the zeolites have been found to be a more robust and physically and chemically stable absorption medium than silica gel, particularly at higher temperatures (e.g. 200 °C) in the typical cyclization reaction solvent systems. Advantageously the zeolite may thus be added at or near the end of the cyclization or at an earlier intermediate stage of the process. The zeolite may even be present in the initial monomer solution prior to its cyclization. Adding the zeolite at the end of the cyclization process is preferred, for example, after a weight fraction cyclic oligomers to total oligomers of 0.6, preferably 0.7, more preferably 0.8, most preferably 0.9 is reached, as then both remaining unreacted monomers as well as linear oligomers are removed.

The cyclic oligomers tend to have a lower solubility in the solvent than the linear oligomers or especially the monomers or other low molecular weight species (less than 100 g/mol) having acidic and/or alcoholic functional groups. Thus the cyclic may additionally or alternatively be removed by cooling the reaction product mixture and/or adding an anti-solvent. Cooling the mixture so that the cyclic species precipitate earlier is often preferred, as most anti-solvents do not have a high selectivity for the cyclic versus linear species.

It is noted that in this application, "optionally-substituted" refers to chemical substituents that are different from hydrogen, alkyl, aryl or alkylaryl groups. Such optional substituents will be generally inert during the ring-closing oligomerization step and may be for example, halogens or ethers.

In the present invention, a "catalyst" refers to an inorganic or metal-containing compound such as an organometallic species or a metal salt; whereas an "organic base" refers to a non-metallic and basic organic species.

In another preferred embodiment of the process, the optional catalyst is either absent or it is present as a metal alkoxide or metal carboxylate, preferably one of tin, zinc, titanium, calcium, iron, aluminium or their mixtures. The lack of a catalyst reduces the cost of raw materials and simplifies the purification and further use of the cyclic polyester oligomer. However some metal-based catalysts have been found by the inventors to be highly effective in the process of the invention thus allowing the cyclic polyester oligomer compositions to be prepared under relatively mild conditions of temperature and time. This then improves productivity and minimizes degradation and discoloration in the process. In addition, in some embodiments, a non-metallic catalyst is used. For example, non-metallic catalysts such as those used for PLA production from lactide may be used. In specific embodiments, the non-metallic catalyst may be selected from one or more of the group consisting of N-heterocyclic carbenes (NHC); tris(pentafluorophenyl)borane (B(C₆F₅)₃); 1,3,4-triphenyl-4,5-dihydro-1H-1,2,4-triazol-5-ylidene carbine; and DMAP/DMAP.HX (where XH=CF₃SO₃H, CH₃SO₃H, HCl, (F₃CSO₂)₂NH) or DMAP/DMAPCH₃.X (where X= I-, -PF₆).

In a specific preferred embodiment of the process, the optional organic base E is present in a stoichiometric ratio of from 0.5 to 6, preferably 1 to 4, more preferably 2 to 3 mol relative to 1 mol of all monomer component species used as a reactant in the process. The use of such optional organic base loading has been found to allow the ring-closing oligomerization to take place under relatively mild conditions of temperature and time while avoiding the catalysis of undesired side reactions during the process. Furthermore contamination is avoided of the polyester oligomer composition product by large quantities of unquenched residual catalysts, which may lead to degradation and/or discoloration in subsequent thermal processing such as polymerization or extrusion or molding of the resulting polymer. Also an effective balance between catalyst cost and productivity is obtained.

In a preferred embodiment of the solvent of the process, the ionic liquid is an ionic liquid in which the cation does not contain acidic protons, more preferably the ionic liquid is N-methyl-N-alkylpyrrolidinium bis(trifluoromethanesulfonyl)imide (PYR_{1R}TFSI).

In another preferred embodiment of the solvent of the process, the optionally-substituted napthalene is selected from the group consisting of naphthalene, 1-methylnaphthalene, and 2-methylnaphthalene.

In still yet another preferred embodiment of the solvent of the process, the optionally-substituted aromatic compound is diphenyl ether, dichlorobenzene, or a xylene, preferably p-xylene.

In a preferred embodiment of the process, the zeolite is selected from either Zeolite Y or Zeolite B and the impurities absorbed comprise one or more linear oligomeric polyester species having furanic and/or alcoholic units. Optionally linear monomeric acid/ol, diacid, diester, or diol species, as well as other low molecular weight (less than 100 g/mol) impurities having acidic or alcoholic groups are preferably also removed. In still a preferred embodiment of the process, the ratio of silica over alumina of the zeolite (SiO2/Al2O3 in mol/mol) is larger than 5, preferably larger than 20, even more preferably larger than 40.

In the sub-steps of subsequent step (III) in which linear oligomeric polyester species having furanic units are separated and removed from the cyclic oligomeric composition the added zeolite and absorbed impurities may be those described above.

The sub-step of cooling of the cyclic oligomeric composition in order to precipitate out cyclic polyester oligomers having furanic units is preferably carried out under temperature and time conditions of 50 to 125 °C and 5 to 180 min, preferably 60 to 110 °C and 30 to 120 min, more preferably 80 to 100 °C and 45 to 90 min.

The sub-step of adding an antisolvent in order to precipitate out cyclic polyester oligomers having furanic units is carried out by adding an antisolvent selected from one or more of hydrocarbons or monoesters in amounts of 5 to 95, preferably 25 to 75, more preferably 30 to 60 weight %. In preferred embodiments, the hydrocarbon is an alkane, preferably hexane, and the ester is a salicylate, preferably a methyl salicylate.

The sub-step of separating zeolites having absorbed impurities from the cyclic oligomeric composition is preferably carried out by means of one or more of the following methods: filtration, sedimentation, and centrifugation. Alternatively, the zeolites are immobilized on a solid support, for example, packed into a column, and they are contacted with the solution by passing the solution as a fluid phase through the column.

The step (III) in which linear oligomeric polyester species having furanic units are separated and removed from the cyclic oligomeric composition may comprise one or more additional separation sub-steps of passing a mobile phase of the cyclic oligomeric composition through a stationary phase, selective precipitation, distillation, extraction, crystallization or their combinations.

Another aspect of the invention concerns a cyclic polyester oligomer composition obtainable, preferably obtained, by a process according to the invention, wherein the composition contains: (i) a residual solvent in a concentration of less than 5, preferably 2, more preferably 1 wt %, and the residual solvent is selected from the group consisting of an ionic liquid, an optionally-substituted napthalene, optionally-substituted aromatic compound, and their mixtures; (ii) linear oligomeric polyester species having furanic units and present in a concentration of less than 5 %, preferably 3, most preferably 1 wt %; and (iii) optionally a zeolite, in a concentration of less than of less than 5, preferably 2, more preferably 1 wt %, wherein the earlier stated weight percentages are relative to the total weight of the cyclic polyester oligomer composition.

The linear oligomeric polyester species having furanic units in the present invention typically contain from 2 to 50, preferably 2 to 20, more preferably 2 to 10 monomeric repeat units (an ester linkage is formed by a reaction of a diacid or diester monomer and a diol in the present invention). The composition containing such low levels of linear species is advantageous in that the subsequent polymerization may be carried out efficiently and reproducibly. Large and/or variable levels of linear species in the cyclic oligomer composition may change the subsequent polymerization stoichiometry and thus affect the obtainable molecular weight upon polymerization. In addition, acidic, alcoholic, or ester end groups of linear oligomeric or monomeric species may react to disadvantageously release volatile species during polymerization. Furthermore reactive acidic species may act to quench the basic catalysts and/or be corrosive to processing equipment.

In a preferred embodiment of the composition, the content of residual monomer components, such as C¹, D¹, C², or D², in the cyclic polyester oligomer composition is less than 5, preferably 3, and most preferably 1 weight percent based on the total weight of the composition.

In one preferred embodiment of the cyclic polyester oligomer composition, the cyclic polyester oligomer composition contains a halogenated impurity, preferably an acid chloride and/or its residue. A residue is defined here as a reaction product or byproduct, for example, a halogen acid such as HCl or a halogen salt such as a chloride salt. Such impurities are a byproduct of the use of acid halide reactants, such as acid chlorides, which have both more favourable kinetics and equilibrium in the production of the oligomer composition than does the reaction of a carboxylic acid with an alcohol. However halogenated species may be corrosive and thus require special expensive construction materials for the subsequent polymerization plant. Therefore their content in the cyclic polyester oligomer composition of the invention will preferably be kept low, e.g. by removal during the subsequent separation and removal step.

In another preferred embodiment of the cyclic polyester oligomer composition, the composition comprises the specific cyclic polyester oligomer having furanic units and of structure Y^{1'} 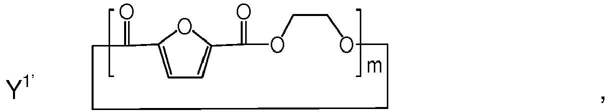 wherein m is an integer from 1 to 20, preferably 2 to 15, most preferably 3 to 10. This embodiment is a suitable raw material for producing poly(2,5-ethylene furandicarboxylate) (PEF), and thus has the advantages previously discussed in relationship to the process to produce this oligomer composition.

In an alternative preferred embodiment of the cyclic polyester oligomer composition, the composition comprises the specific cyclic polyester oligomer having furanic units and of structure Y^{1"} 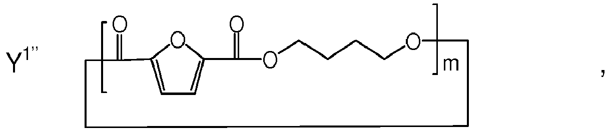 wherein m is an integer from 1 to 20, preferably 2 to 15, most preferably 3 to 10. This embodiment is a suitable raw material for producing poly(2,5-ethylene furandicarboxylate) (PBF), and thus has the advantages previously discussed in relationship to the process to produce this oligomer composition.

Another aspect of the invention is a process to produce a polyester polymer comprising (i) the process of the invention to prepare a cyclic oligomer composition comprising a cyclic polyester oligomer having furanic units together with (ii) a subsequent polymerization step to produce a polyester polymer.

In a preferred embodiment of the process, the subsequent polymerization is carried out in the presence of a plasticizer. In an alternative embodiment of this aspect of the invention, the subsequent polymerization in the presence of a plasticizer is carried out on a cyclic oligomer composition obtainable, preferably obtained, by processes known in the art, preferably as disclosed in the earlier discussed WO2014/139603.

According to the present invention, a plasticizer is a compound which is capable of decreasing the melting point and/or the viscosity of a cyclic polyester oligomer having furanic units, preferably a cyclic dimer. In preferred embodiments, the amount of plasticizer during the polymerization is sufficient to reduce the melting point of the initial unreacted cyclic oligomer composition by at least 10, preferably 25, more preferably 50, most preferably about 75 °C. In preferred embodiments, the amount of plasticizer during the polymerization is sufficient to reduce the initial melt viscosity of the reaction mixture comprising the cyclic oligomer composition by at least 10, preferably 25, more preferably 50 %. In certain other preferred embodiments, the amount of plasticizer is from 1 to 75, preferably 5 to 60, more preferably 10 to 50 mass, most preferably 15 to 40 mass % based on the mass of the initial cyclic polyester oligomer composition. The melt viscosity may be measured using a rheometer according to methods commonly known in the art. In a specifically preferred embodiment, the plasticizer is one or more selected from the group consisting of a supercritical fluid, an optionally-substituted phenyl ether, an ionic liquid, an optionally-substituted xylene, a polyether, and their mixtures. In a more specifically preferred embodiment of the process, the supercritical fluid is carbon dioxide or the polyether is a glyme, preferably tetraethylene glycol dimethyl ether. In an alternative embodiment, the plasticizer is a polyester polymer of the cyclic polyester oligomer, preferably a PEF polymer, preferably one having a molecular weight of about 15, 000 to 30,000 g/mol (melting point about 220 °C), or a PEF oligomer, preferably one having a molecular weight of about 1,000 to less than about 15,000 g/mol. The previously stated molecular weights are measured by SEC relative to polystyrene standards. In yet another preferred embodiment, mixtures of any of the above-mentioned plasticizers are used.

The presence of the plasticizer has several beneficial effects. First of all, some of the cyclic species, such as the cyclic dimer, have quite high melting points, which the presence of the plasticizer then effectively reduces. Thus most cyclic oligomer compositions will not melt at preferred temperatures below 300 C, above which the thermal degradation of both the oligomers and their polymer product cause significant colour formation and reduction of the achieved molecular weight in the polymer product. The plasticizer beneficially minimizes then the thermal profile (temperature and/or time) of the reactant cyclic oligomer composition during the initial melt up and any holding of the composition prior to its melt polymerization. In addition, the plasticizer facilitates the polymerization of the cyclic oligomer compositions at temperatures well below their melting point and thus under milder conditions.

Furthermore many of the desirable polymerization catalysts are solid rather than liquids. Examples include cyclic stannoxane, which readily yields high molecular weight polymers for commercial applications, as well as the metal oxides such as Sb₂O₃ and Bi₂O₃, which provide polymer products having reduced colour versus that obtainable with tin-based catalysts. The inert plasticizer facilitates the polymerization by promoting the intimate contacting and mixing between the cyclic oligomer compositions and these solid state polymerization catalysts.

In another aspect of the invention, the ring-opening polymerization in the presence of a plasticizer as described above and disclosed in this application may also be carried out on known cyclic oligomer compositions comprising a cyclic polyester oligomer having furanic units, such as those disclosed in EP2931784 (A1).

In yet another aspect of the ring-opening polymerizations in the presence of a plasticizer as described above and disclosed in this application, the polymerization is carried out in the presence of one or more anti-oxidants such as substituted phenols and derivatives of phenylenediamine. Suitable anti-oxidants include IRGANOX 1098, which is the trade name for benzenepropanamide, N,N'-1,6-hexanediylbis[3,5-bis(1,1-dimethylethyl)-4-hydroxy; a sterically hindered phenolic antioxidant such as IRGANOX 1076, which has the formula Octadecyl-3-(3,5-di-tert.butyl-4-hydroxyphenyl)-propionate,

A related aspect of the invention is the use of the cyclic polyester oligomer composition of the invention in the production of a polyester polymer. This polymerization process and use advantageously utilize the desirable properties of the oligomer composition as a raw material in a polymerization process, such as the favourable kinetics, lack of corrosive acidic species, and lack of formation of significant quantities of volatile species during the polymerization.

One skilled in the art will understand that the combination of the subject matters of the various claims and embodiments of the invention is possible without limitation in the invention to the extent that such combinations are technically feasible. In this combination, the subject matter of any one claim may be combined with the subject matter of one or more of the other claims. In this combination of subject matters, the subject matter of any one process claim may be combined with the subject matter of one or more other process claims or the subject matter of one or more composition claims or the subject matter of a mixture of one or more process claims and composition claims. By analogy, the subject matter of any one composition claim may be combined with the subject matter of one or more other composition claims or the subject matter of one or more process claims or the subject matter of a mixture of one or more process claims and system claims. One skilled in the art will also understand that the combination of the subject matters of the various embodiments of the invention is also possible without limitation in the invention to the extent that such combinations are technically feasible.

### BRIEF DESCRIPTION OF THE DRAWINGS

The invention will be explained in more detail hereinafter with reference to various embodiments of the invention as well as to the drawings. The schematic drawings show:
- Fig. 1: shows a reaction scheme for the synthesis of a cyclic polyester oligomer having furanic units of structure Y¹ from the reaction of a monomer component C¹ or D¹ in a ring closing oligomerization step.
- Fig. 2: shows a reaction scheme for the synthesis of a cyclic polyester oligomer having furanic units of structure Y² from the reaction of a monomer component C² or D² in a ring closing oligomerization step.
- Fig. 3: shows a reaction scheme for the synthesis of a specific cyclic polyester oligomer useful in the production of PEF and having furanic units and of structure Y^{1'} from the reaction of a specific monomer component C^{1'} or D¹ in a ring closing oligomerization step.
- Fig. 4: shows a reaction scheme for the synthesis of a specific cyclic polyester oligomer useful in the production of PBF and having furanic units and of structure Y^{1"} from the reaction of a specific monomer component C^{1"} or D^{1"} in a ring closing oligomerization step.
- Fig. 5: shows the effect of plasticization on the conversion of the cyclic PEF dimer during ring-opening polymerization.

### DETAILED DESCRIPTION OF THE INVENTION

The claimed invention relates to a process to prepare a cyclic polyester oligomer composition comprising a cyclic polyester oligomer having furanic units, such as that known from WO2014/139603 (A1), which is hereby incorporated by reference.

The cyclic polyester oligomer composition of the current invention is not specifically limited and it may comprise other components in addition to the polyester polymer having furanic units and comprising the structure Y¹ or Y². For example, the cyclic polyester oligomer composition may additionally comprise small amounts of one or more unreacted and/or unremoved reaction components such as a monomer component (unreacted diacid, diol, or acidol reagents), a catalyst, a templating agent, a base, a catalyst quencher, a solvent, used in the preparation of the cyclic polyester oligomer. The amount of these impurities in the cyclic polyester oligomer will preferably be less than 10, more preferably less than 5, even more preferably less than 3, and most preferably less than 1 weight % based on the total weight of the cyclic polyester oligomer.

In addition, the cyclic polyester oligomer composition may additionally comprise low levels of impurities introduced as a contaminant in one of the reaction components or formed due to a side reaction during the ring-closing oligomerization step or an optional additional step such as a subsequent devolatization step. Examples of such impurities are linear oligomeric polyester species having furanic units. Finally the cyclic polyester oligomer composition may additionally comprise additional components such as typical monomer additives added during production or prior to use such as stabilizers against oxidation, thermal degradation, light or UV radiation. One skilled in the art will understand that blends with other monomers in order to combine the favorable properties of different monomers are also contemplated as being within the scope of the present invention.

In one embodiment, the content of diacid, diol, or acidol monomers in the cyclic polyester oligomer composition is less than 5 wt %, preferably less than 3 wt %, more preferably less than 1 wt %. In the present application, the content of diacid, diol, or acidol monomers refers to their content as measured by the extraction of soluble species followed by GC-MS analysis.

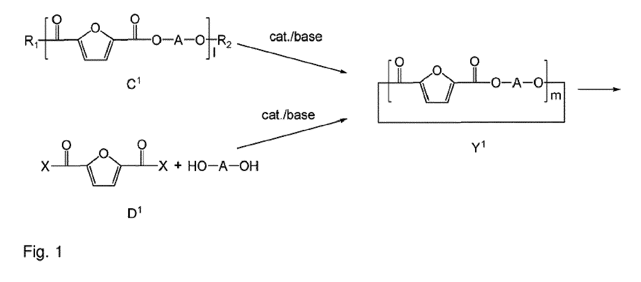

As shown in Figure 1, the process of the invention to prepare the cyclic oligomer composition comprising a cyclic polyester oligomer of structure Y¹ having furanic units comprises the step of (I) reacting a monomer component C¹ or D¹ in the presence of an optional catalyst and/or optional organic base in a ring closing oligomerization step under conditions of a reaction temperature and reaction time sufficient to yield a cyclic polyester oligomer having furanic units of structure Y¹, wherein the monomer component C¹ comprises the structure 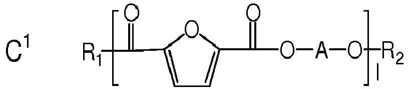 and wherein each of the groups A is an optionally-substituted linear, branched or cyclic alkyl, phenyl, aryl, or alkylaryl, and wherein I is an integer from 1 to 100, preferably 2 to 50, most preferably 3 to 25, and wherein
R₁= OH, OR, halogen, or O-A-OH,
R = optionally substituted linear, branched or cyclic alkyl, phenyl, aryl or alkylaryl,
R₂= H or 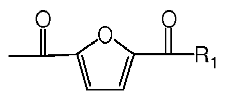 wherein the monomer component D¹ comprises the structures 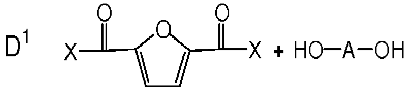 and wherein A is an optionally-substituted linear, branched or cyclic alkyl, phenyl, aryl, or alkylaryl, and wherein each of the groups X is an OH, a halogen, or an optionally-substituted alkyloxy, phenoxy, or aryloxy, and wherein the groups X are not OH when A is n-butyl.

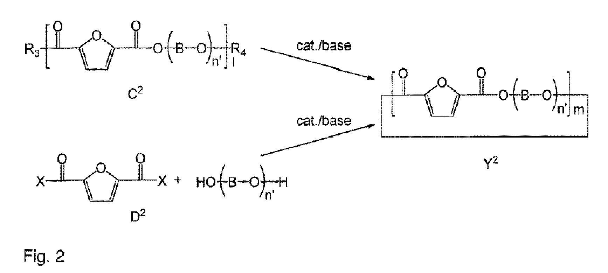

As shown in Figure 2, the process of the invention to prepare the cyclic oligomer composition comprising a cyclic polyester oligomer of structure Y² having furanic units comprises the step of (II) reacting a monomer component C² or D² in the presence of an optional catalyst and/or optional organic base in a ring closing oligomerization step under conditions of a reaction temperature and reaction time sufficient to yield a cyclic polyester oligomer having furanic units of structure Y², wherein the monomer component C² comprises the structure 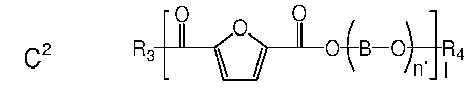 and wherein each of the groups B is an optionally-substituted linear, branched or cyclic alkyl, phenyl, aryl, or alkylaryl, wherein I is an integer as defined above, and wherein n' is an integer from 1 to 20, preferably 2 to 10, and wherein
R₃= OH, OR, halogen, or O-(B-O)ₙ-H,
R = optionally substituted linear, branched or cyclic alkyl, phenyl, aryl or alkylaryl,
R₄= H or 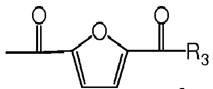 the monomer component D² comprises the structure 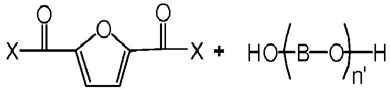 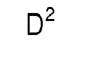 and wherein each of the groups X is an OH, a halogen, or an optionally-substituted alkyloxy, phenoxy, or aryloxy, each of the groups B is an optionally-substituted linear, branched or cyclic alkyl, phenyl, aryl, or alkylaryl, and n' is an integer as defined previously for Y².

In a step (III) subsequent to either (I) or (II), linear oligomeric polyester species having furanic units are separated and removed from the cyclic oligomeric composition.

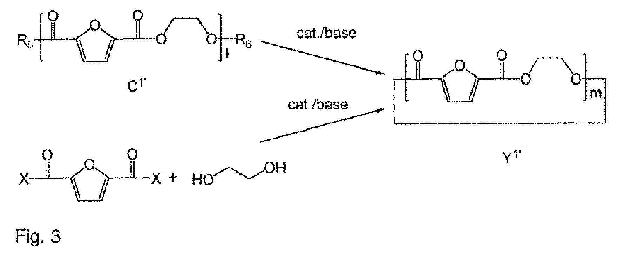

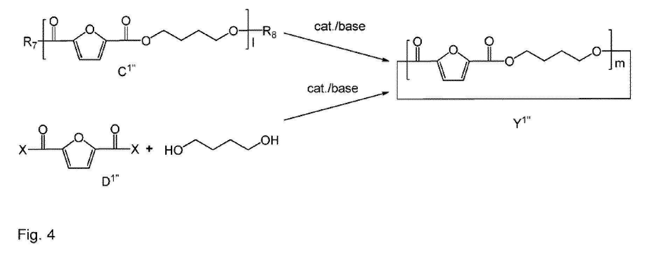

Fig. 3 shows a reaction scheme for the synthesis of a specific cyclic polyester oligomer useful in the production of PEF and having furanic units and of structure Y^{1'} from the reaction of a specific monomer component C^{1'} or D^{1'} in a ring closing oligomerization step, and Fig. 4 shows a reaction scheme for the synthesis of a specific cyclic polyester oligomer useful in the production of PBF and having furanic units and of structure Y^{1"} from the reaction of a specific monomer component C^{1"} or D^{1"} in a ring closing oligomerization step, wherein I, m and n are as previously defined for the case of both figures.

Unless specifically indicated otherwise, conventional ring-closing oligomerization processes and their various reagents, operating parameters and conditions, such as that known from WO2014/139603 (A1), may be used in the processes according to the invention in preparing the cyclic polyester oligomers having the structures Y¹, Y², Y^{1'}, or Y^{1"}.

The conditions of a reaction temperature and reaction time sufficient to yield a cyclic polyester oligomer having furanic units in the ring-closing oligomerization step are not specifically limited. Sufficient here means that the reaction temperature and time are sufficient to cause a ring-closing reaction to occur such that an oligomer having the claimed values of m is produced from the monomer components. One skilled in the art will understand that appropriate specific reaction temperatures and reaction times may vary somewhat due to the interaction between the reaction temperature and time.

For example, increasing the reaction temperature may allow the reaction to take place in a shorter time, or increasing the reaction time may allow lower reaction temperatures to be used. Lower reaction temperatures and/or shorter reaction times may be appropriate if a lower molecular weight cyclic polyester oligomer is to be produced and/or a lower conversion of monomer component to oligomer may be tolerated. Alternatively, higher reaction temperatures and/or longer reaction times may be appropriate if a higher molecular weight cyclic polyester oligomer is to be produced and/or a higher conversion of monomer component is desired.

Furthermore the use of more effective catalysts or bases or a higher concentration of catalyst or organic base may allow milder reaction conditions (e.g. lower reaction temperatures and shorter reaction times) to be used. Conversely the presence of impurities, particularly catalyst-quenching or chain-stopping impurities may require more intensive reaction conditions.

In one embodiment the cyclization reaction temperature is from 100 to 350, preferably 150 to 300, most preferably 180 to 280 °C, and the reaction time is from 30 to 600, preferably 40 to 400, most preferably 50 to 300 minutes. In certain specific embodiments, the various specific temperature and time range combinations obtained by combining any of these disclosed ranges may be used. In a more preferred embodiment, these temperature and/or time ranges are used in the ring closing oligomerization step with monomer components C¹ or C².

In another embodiment the cyclization reaction temperature is from -10 to 150, preferably -5 to 100, most preferably 0 to 80 °C, and the reaction time is from 5 to 240, preferably 10 to 180, most preferably 15 to 120 minutes. In certain specific embodiments, the various specific temperature and time range combinations obtained by combining any of these disclosed ranges may be used. In a more preferred embodiment, these temperature and/or time ranges are used in the ring closing oligomerization step with monomer components D¹ or D².

In the execution of the present invention, any catalyst which is able to catalyze the ring-closing oligomerization to form cyclic polyester oligomers may be used. Suitable catalysts for use in the present invention are those known in the art for polymerization of cyclic esters, such as an inorganic base, preferably a metal alkoxide, a metal carboxylate, or a Lewis acid catalyst. The Lewis acid catalyst may be a metal coordination compound comprising a metal ion having more than one stable oxidation state. Of this class of catalysts, the tin-or zinc-containing compounds are preferred, of which their alkoxides and carboxylates are more preferred, and tin octoate is the most preferred catalyst.

The ring-closing oligomerization step preferably takes place in the presence of an optional organic base. The organic base is not specifically limited, and, it may be an inorganic or organic base. In one embodiment, it has the general structure E, and in other embodiments it is an alkyl amine such as triethylamine or it is pyridine. In still other embodiments, it is a combination of E and an alkyl amine. In this application, a "catalyst" refers to an inorganic or metal-containing compound such as an organometallic species or a metal salt; whereas an "organic base" refers to a non-metallic and basic organic species.

Specific combinations of catalysts and bases may be particularly effective, and their use may be preferred. In one preferred embodiment, the catalyst is a tin, zinc, titanium, or aluminum alkoxide or carboxylate, and the organic base is DABCO (CAS No. 280-57-9) or DBU (CAS No. 83329-50-4), preferably together with triethyl-amine. The monomer component may be in the solid phase when it is mixed with the catalyst and/or organic base. However, bringing the monomer component into the molten phase or a liquid phase using a solvent and then adding the catalyst and/or organic base afterwards is preferred.

The amount of catalyst and/or organic base in the process of the invention is not specifically limited. In general, the amount of catalyst and/or organic base is sufficient to cause a ring-closing oligomerization step to occur for the selected reaction temperature and time such that an oligomer having the claimed values of I is produced from the monomer components. In one embodiment, the catalyst and/or organic base is present, and the catalyst is present in an amount relative to the total weight of the monomer components of from 1 ppm to 1 weight%, preferably from 10 to 1,000 ppm, more preferably from 50 to 500 ppm, and the organic base is present in a stoichiometric ratio of from 0.5 to 6, preferably 1 to 4, more preferably 2 to 3 mol relative to 1 mol of all monomer component species used as a reactant in the process. The concentration of the catalyst and the organic base may be readily determined by the masses or mass flow rates used of these reagents relative to that of the monomer components.

The process to prepare the cyclic polyester oligomer composition of the invention is not specifically limited, and it may be conducted in a batch, semi-continuous, or continuous manner. Oligomerization processes suitable for preparing the cyclic polyester oligomer composition of the invention can be divided into two groups, solution oligomerization in the presence of a solvent, or oligomerization in the substantial absence of solvent, e.g., melt oligomerization, carried out at a temperature above the melting temperature of the monomer components and oligomeric species.

As the presence of substantial amounts of unreacted monomer component, linear oligomers, or other low molecular weight species, particularly those having acidic or other free OH groups, in the cyclic polyester oligomer composition may detrimentally affect the storage stability and/or polymerization processing behaviour of the oligomer composition, the cyclic polyester oligomer composition is subjected to a step in which linear oligomeric polyester species, as well as optionally other impurities, such as low molecular weight (e.g. less than 100 g/mol) species having acidic and/or hydroxyl groups, are removed.

The step in which linear oligomeric polyester species having furanic units, as well as optionally other impurities, are separated and removed from the cyclic polyester oligomer composition of the invention is not specifically limited. Examples of other impurities may be unreacted starting materials such as diacids or diols or residual reagents such as bases or their residues (e.g. amine residues). Separation and purification methods are well-known in the art, for example, as disclosed in Purification of Laboratory Chemicals, Sixth Ed., by W.E. Armarego and C.L.L. Chai, published in 2009 by Elsevier, Oxford (ISBN-13: 978-1856175678), and The Molecular World, Separation, Purification and Identification by L.E. Smart, published in 2002 by the Royal Society of Chemistry, Cambridge (ISBN: 978-1-84755-783-4).

Unless specifically indicated otherwise, conventional separation and purification processes and their various apparatuses, operating parameters and conditions may be used in the processes according to the invention in preparing the cyclic polyester oligomers of structures Y¹, Y², Y^{1'}, or Y^{1"} and their compositions.

In one embodiment the separation step in which linear oligomeric species and optionally other impurities are removed comprises one or more separation sub-steps of passing a mobile phase of the cyclic oligomeric composition through a stationary phase, selective precipitation, distillation, extraction, crystallization or their combinations.

In the cyclic polyester oligomer composition product that is obtained after the separation step, linear oligomeric polyester species having furanic units are generally present in an amount of less than 5 wt. %, more in particular in an amount of less than 3 wt. %, still more in particular in an amount of less than 1 wt.% relative to the total weight of the cyclic polyester oligomer composition. The content of linear oligomeric polyester species having furanic units in the cyclic polyester oligomer composition of the invention may be readily determined by conventional methods. For example, the content of linear oligomeric species may be determined by electrospray mass spectrometry, matrix-assisted laser desorption/ ionization (MALDI) mass spectrometry, high-performance liquid chromatography (HPLC) method coupled to mass spectronomy, and gel filtration chromatography. In the present application and invention, the concentration of linear oligomeric polyester species having furanic units refers to the concentration as determined by HPLC.

In a preferred embodiment of the composition, the content of residual monomer components, such as C¹, D¹, C², or D², in the cyclic polyester oligomer composition is less than 5, preferably 3, and most preferably 1 weight percent based on the total weight of the composition. The content of such residual monomer (or solvent) components may be determined by FTIR or NMR spectroscopic analysis of the composition. Alternatively the content may be determined by chromatographic methods such as HPLC or GC. In the present application and invention, the concentration of residual monomer (and solvent) components refers to the concentration as determined by HPLC.

The invention relates to a cyclic polyester oligomer composition comprising a cyclic polyester oligomer having furanic units, wherein the structure of the cyclic polyester oligomer having furanic units is Y¹ or Y², and wherein the polyester polymer composition is obtainable with the above-described method. Said cyclic polyester oligomer composition is characterized in that the composition contains: (i) a residual solvent in a concentration of less than 5, preferably 2, more preferably 1 wt %, and selected from the group consisting of an ionic liquid, an optionally-substituted napthalene, optionally-substituted aromatic compound, and their mixtures, (ii) linear oligomeric polyester species having furanic units and present in a concentration of less than 5 %, preferably 3, most preferably 1 wt %, and (iii) optionally a zeolite, in a concentration of less than of less than 5, preferably 2, more preferably 1 wt %, wherein the weight percentages are relative to the total weight of the cyclic polyester oligomer composition. Such oligomer compositions can answer most requirements posed by the current polymerization applications.

In another preferred embodiment, the composition comprises a halogenated impurity, preferably an acid chloride and/or its residue. Methods of detection of halogenated impurities in oligomers are well-known and include combustion ion chromatography (IC), optical atomic spectroscopy, and X-ray fluorescence analysis (XRF). However halogenated species may be corrosive and thus require special expensive construction materials for the subsequent polymerization plant. Therefore their content in the cyclic polyester oligomer composition of the invention will preferably be kept low, e.g. by removal during the subsequent separation and removal step.

In a preferred embodiment of the cyclic polyester oligomer composition, the specific cyclic polyester oligomer having furanic units is one of structure Y^{1'} or Y^{1"}, wherein m is an integer from 1 to 20, preferably 2 to 15, most preferably 3 to 10.

Yet another aspect of the present invention is a process to produce a polyester polymer comprising (i) the process of the invention to prepare a cyclic oligomer composition comprising a cyclic polyester oligomer having furanic units together with (ii) a subsequent polymerization step to produce a polyester polymer. Suitable ring opening polymerization catalysts, process conditions, apparatuses and methods are those disclosed in the earlier discussed WO2014/139602, which is hereby incorporated by reference. Related to this aspect is the aspect of the use of the cyclic polyester oligomer composition of the invention in the production of a polyester polymer. Preferred embodiments of this process or use are those in which the polyester polymer is a PEF polymer or a PBF polymer.

Particularly preferred is a polyester polymer composition obtainable, preferably obtained, by the ring opening polymerization process of the invention, wherein the composition contains: (i) a plasticizer selected from the group consisting of an optionally-substituted phenyl ether, an ionic liquid, an optionally-substituted xylene, a polyether, and their mixtures, (ii) a cyclic polyester oligomer having furanic units, and (iii) EITHER: (a) a PEF polymer OR (b) a PBF polymer. The residual plasticizer is preferably present in an amount of less than 10, more preferably 5, even more preferably 2, and most preferably 1 wt %. The content of plasticizer in the polymer may be measured by conventional methods such as that disclosed in Quantifying Polymer Plasticizer Content Through Direct Analysis of Tracer Compounds, IP.com Disclosure Number: IPCOM000246667D, Publication Date: 2016-Jun-24. The residual unreacted cyclic polyester oligomer having furanic units is preferably present in an amount of less than 5, more preferably 2, even more preferably 1 wt %. In some embodiments, the content of residual plasticizer and unreacted cyclic oligomer is measured by means of their separation from the polymer via solvent extraction, high temperature distillation or column chromatography and then followed by their identification by means of UV, NMR, or IR spectroscopies and/or mass spectrometry. The PEF and PBF polymers will often preferably have molecular weights of at least 10,000, preferably 15,000, more preferably 20,000 Daltons relative to polystyrene standards as measured by SEC.

### EXAMPLES

The following examples are set forth to provide those of ordinary skill in the art with a detailed description of how the processes, polyester polymer compositions, and uses claimed herein are evaluated, and they are not intended to limit the scope of what the inventors regard as their invention.

In these examples, the following characterization methods are parameters were used for the characterization of the cyclic polyester oligomer compositions prepared in the examples.

### SEC-MALS

Conversion and Molecular weight distributions of the polyesters were analyzed using size exclusion chromatography coupled with multi-angle light scattering (SEC-MALS) on an Agilent 1100 GPC using two PFG linear M columns (PSS) connected in series with an Agilent 1100 VWD/UV detector operated at 290 nm, a DAWN HELEOS II multi-angle laser light scattering (MALS) detector (Wyatt Technology Europe) followed by an Agilent 1100 RI detector. Samples were eluted in HFIP with 0.02 M K-TFAc at 1 mL/min at room temperature.

### ¹H NMR

Measurements were made on a Bruker AV 300 spectrometer operating at a frequency of 300 MHz and using CDCl₃ as solvent.

### HPLC-MS

An Agilent 1200 Series HPLC with a quaternary pump, autosampler and UV detector was equipped with an Agilent Eclipse XDB-C18, 5 m, 4.6x150 mm column. The eluent mixture was composed of (A) Water stabilized with formic acid (1mL/L) and (B) Acetonitrile stabilized with formic acid (1mL/L). A gradient was run at 1 mL/min for 60 min. Solvent ratio of B was changed linearly from 20 % to 45.2 % during 11 min, then from 45.2 % to 80 % during 29 min, followed by 10 minutes at 97 % and 10 minutes at 20 %. Samples were dissolved at 1 mg/mL in HFIP/CHCL3 (15%). Injection volume was 10 µL and UV detection was carried out at 280 nm. Peaks were characterized by online-mass spectroscopy with an Agilent 1640 single-quadrupol MS.

### MALDI-TOF

The matrix was T-2-[3-(4-t-Butyl-phenyl)-2-methyl-2-propenylidene]malononitrile (DCTB) +Na Mix 10:1, and the instrument type was a Bruker Daltonics Ultraflex II, and the acquisition mode was reflector.

### Example 1: A Cyclic Polyester Oligomer Composition (Embodiment Of Y^{1'})

### For Production Of PEF

In this example, the preparation is described of the cyclic polyester oligomer shown in FIG. 3, which may then subsequently be used to prepare PEF, poly(2,5-ethylene furandicarboxylate). 40 g of me-FDCA were charged together with 20 mL of EG into a glass reactor equipped with a stirrer. The reaction was carried out under inert atmosphere at a starting temperature of 140 °C in the presence of 0.50 g catalyst (Bu2SnO) and progressively heated to a final temperature 180 °C. After 1 hour of reaction pressure was reduced to 700 mbar; pressure was reduced again after 40 minutes to 400 mbar and further to 200 mbar after 30 minutes. Finally the pressure was stepwise reduced until 10 mbar. Temperature was increased up to 200°C and the system was left under this condition for 2 hours. The system was allowed to cool to room temperature and the solid product was removed, ground, and dried. The pre-polymer obtained was characterized with HPLC and GPC, and its identity was confirmed to be C¹'.

The pre-polymer C¹' was dissolved in 2-methylnaphthalene as solvent at a concentration of 10 g/l, and the resulting solution was reacted under inert atmosphere at 200 °C (in the absence of added additional catalyst) for 3 hours in order to transform the pre-polymer C¹' into the cyclic oligomer Y¹'. Next Zeolite Y was added at a concentration of 10g/l. HPLC analysis confirmed that the concentrations of the cyclic oligomers (m = 2 to 5) remained essentially unchanged, but that the linear species (I = 1 to 8) were essentially removed from the solution. This result confirms that unreacted linear residual species can be easily removed from the reaction system by adsorption on zeolites.

### Comparative Example 1 and 2: Lack of Polymerization of Cyclic Polyester Oligomer Composition (Y^{1'}) In the Presence of Low Amount of Plasticizer Without Catalyst or Without Catalyst or Plasticizer

In this example, the cyclic oligomer Y1' (m = 2) of Example 1 was reacted for 30 min each at different temperatures between 260 °C and 320 °C with tetra-glyme as plasticizer at a concentration of 60 uL tetra-glyme per 180 mg of cyclic oligomer Y1' and in the absence of added catalyst under inert atmosphere. No reaction occurred and the material remained unchanged.

In a second comparative example, a mixed cyclic oligomer Y1' (m = 2 to 7) of Example 1 was reacted at a temperature of 280 °C for 60 min without added plasticizer or catalyst. GPC analysis confirmed that no reaction of the m = 2 cyclic oligomer occurred. Therefore these comparative examples show that the typically most-abundant species, the low Mw cyclic oligomers (m = 2), will not polymerize in the absence of catalyst or plasticizer.

### Example 2: Production of PEF From Cyclic Polyester Oligomer Composition ( Y^{1'}): In the Presence of Low Amount of Plasticizer With Catalyst

In this example, the cyclic oligomer Y1' (m = 2) of Example 1 was reacted as in the Comparative Example 1, but in the presence of cyclic stannoxane as catalyst in a concentration of 0.1mol% per mol cyclic oligomer repeat units. In this case, a conversion of greater than 95 % was achieved within 20 min.

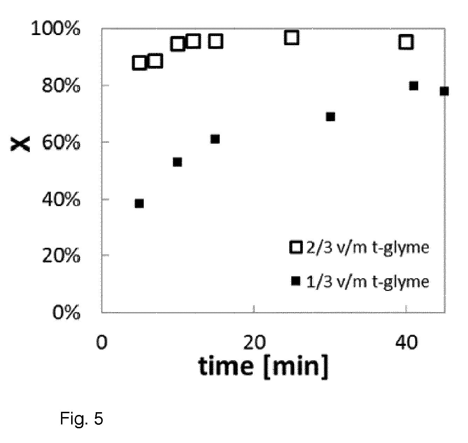

Fig. 5 shows comparative data for the conversion of the cyclic PEF dimer with both a lower (1/3 v/m) and a higher (2/3 v/m) concentration of the tetra-glyme plasticizer.

In other runs of the polymerization, other metal oxide catalysts such as Sb₂O₃ or Bi₂O₃ were compared to tin-based catalysts. It was observed that the polymers prepared using the Sb₂O₃ or Bi₂O₃ were more water-white in appearance than the somewhat yellowish-brown colour obtained with the tin-based catalysts.

### Example 3: Production of PEF From Cyclic Polyester Oligomer Composition ( Y^{1'}): In the Presence of Higher Amount of Plasticizer Without Catalyst

In this example, the cyclic oligomer Y1' (m = 2) of Example 1 was reacted as in the Comparative Example 1, with tetra-glyme as plasticizer at a higher concentration of 240 uL tetra-glyme per 180 mg of cyclic oligomer Y1'. In this case, a conversion of greater than 95 % was achieved within 60 min at all temperatures.

While various embodiments have been set forth for the purpose of illustration, the foregoing descriptions should not be deemed to be a limitation on the scope herein. Accordingly, various modifications, adaptations, and alternatives can occur to one skilled in the art without departing from the spirit and scope herein.

## Claims

1. a process to prepare a cyclic polyester oligomer composition comprising a cyclic polyester oligomer having furanic units, wherein the process comprises:
- a step of either:
(I) reacting a monomer component C¹ or D¹ in the presence of an optional catalyst and/or optional organic base in a ring closing oligomerization step under conditions of a reaction temperature and reaction time sufficient to yield a cyclic polyester oligomer having furanic units and of structure Y¹, wherein the monomer component C¹ comprises the structure 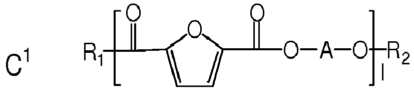 and wherein each of the groups A is an optionally-substituted linear, branched or cyclic alkyl, phenyl, aryl, or alkylaryl, and wherein I is an integer from 1 to 100, preferably 2 to 50, most preferably 3 to 25,
and wherein
R₁= OH, OR, halogen, or O-A-OH,
R = optionally substituted linear, branched or cyclic alkyl, phenyl, aryl or alkylaryl, 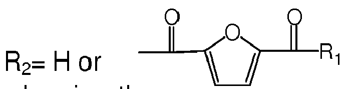
wherein the monomer component D¹ comprises the structure 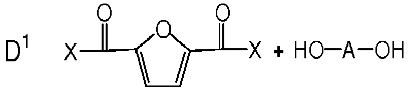 and wherein A is an optionally-substituted linear, branched or cyclic alkyl, phenyl, aryl, or alkylaryl, and wherein each of the groups X is an OH, a halogen, or an optionally-substituted alkyloxy, phenoxy, or aryloxy, and wherein the groups X are not OH when A is n-butyl, and wherein the structure Y¹ of the cyclic polyester oligomer having furanic units is 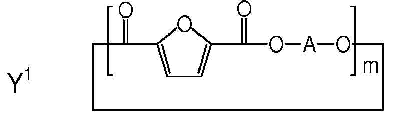 wherein m is an integer from 1 to 20, preferably 2 to 15, most preferably 3 to 10,
OR
(II) reacting a monomer component C² or D² in the presence of an optional catalyst and/or optional organic base in a ring closing oligomerization step under conditions of a reaction temperature and reaction time sufficient to yield a cyclic polyester oligomer having furanic units and of structure Y², wherein the monomer component C² comprises the structure 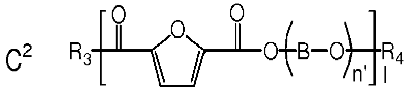 and wherein each of the groups B is an optionally-substituted linear, branched or cyclic alkyl, phenyl, aryl, or alkylaryl, wherein I is an integer as defined above, and wherein n' is an integer from 1 to 20, preferably 2 to 10, and wherein
R₃= OH, OR, halogen, or O-(B-O)ₙ-H,
R = optionally substituted linear, branched or cyclic alkyl, phenyl, aryl or alkylaryl,
R₄= H or 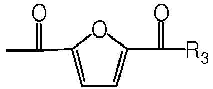 the monomer component D² comprises the structures 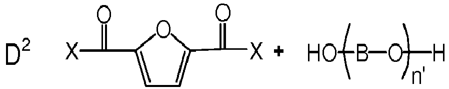 and wherein each of the groups X is an OH, a halogen, or an optionally-substituted alkyloxy, phenoxy, or aryloxy, each of the groups B is an optionally-substituted linear, branched or cyclic alkyl, phenyl, aryl, or alkylaryl, and n' is an integer as defined above, and wherein the structure Y² of the cyclic polyester oligomer having furanic units is 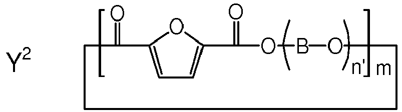 wherein each of the groups B is an optionally-substituted linear, branched or cyclic alkyl, phenyl, aryl, or alkylaryl, n' is an integer as defined above, and m is an integer from 1 to 20, preferably 2 to 15, most preferably 3 to 10,
AND
- a subsequent step (III) in which linear oligomeric polyester species having furanic units are separated and removed from the cyclic oligomeric composition,
**characterized in that**
- the reacting of the monomer component C1 or D1 or C² or D² in the presence of an optional catalyst and/or optional organic base in a ring closing oligomerization step is carried out in the presence of a solvent, wherein the solvent is selected from the group consisting of an ionic liquid, an optionally-substituted napthalene, optionally-substituted aromatic compound, and their mixtures, and optionally in the presence of a zeolite and absorbing impurities onto the zeolite,
- and the subsequent step (III) in which linear oligomeric polyester species having furanic units are separated and removed from the cyclic oligomeric composition comprises one or more of the following sub-steps:
- adding a zeolite and absorbing impurities onto the zeolite,
- cooling the cyclic oligomeric composition in order to precipitate out cyclic polyester oligomers having furanic units,
- adding an antisolvent in order to precipitate out cyclic polyester oligomers having furanic units,
- separating zeolites having absorbed impurities from the cyclic oligomeric composition.

2. The process of claim 1, wherein either:
(I) - the monomer component is C¹ and A is an optionally-substituted linear, branched or cyclic alkyl, I is an integer from 3 to 25, and m is an integer from 3 to 10,
OR
- the monomer component is D¹ and A is an optionally-substituted linear, branched or cyclic alkyl, X is a halogen, or optionally-substituted alkyloxy or phenoxy, and m is as defined previously in this claim, and wherein the structure of the cyclic polyester oligomer having furanic units is one of Y¹,
OR
(II) - the monomer component is C² and wherein B is an optionally-substituted linear, branched or cyclic alkyl, I and m are integers as defined above, and n' is an integer from 2 to 10, OR
- the monomer component is D², and wherein X is an OH, a halogen, or optionally-substituted alkyloxy, phenoxy, or aryloxy, B is an optionally-substituted linear, branched or cyclic alkyl, or phenyl, and n' and m are integers as defined previously in this claim, and wherein the structure of the cyclic polyester oligomer having furanic units is one of Y².

3. The process of either one of claims 1 to 2, wherein either
- the monomer component is C¹ and A is an optionally-substituted linear, branched or cyclic C₁ to C₆ alkyl, and I is an integer from 3 to 25, and m is an integer from 3 to 10,
- the monomer component is D¹ and A is an optionally-substituted linear, branched or cyclic C₁ to C₆ alkyl, X is a halogen, or optionally-substituted alkyloxy or phenoxy, and m is an integer as defined above,
- the monomer component is C² and wherein B is an optionally-substituted linear, branched or cyclic C₁ to C₆ alkyl, I and m are integers as defined above and n' is an integer from 2 to 10, OR
- the monomer component is D², X is a halogen, or an optionally-substituted alkyloxy, phenoxy, or aryloxy, B is an optionally-substituted linear, branched or cyclic C₁ to C₆ alkyl, or phenyl, and n' and m are integers as defined in claim 2.

4. The process of any one of claims 1 to 3, wherein the monomer component is C¹ or C² and the reaction temperature is from 100 to 350, preferably 150 to 300, most preferably 180 to 280 °C, and wherein the reaction time is from 30 to 600, preferably 40 to 400, most preferably 50 to 300 minutes, OR
wherein the monomer component is D¹ or D² and the reaction temperature is from -10 to 150, preferably -5 to 100, most preferably 0 to 80 °C, and wherein the reaction time is from 5 to 240, preferably 10 to 180, most preferably 15 to 120 minutes.

5. The process of any one of claims 1 to 4, wherein either the monomer component C¹ comprises the specific structure 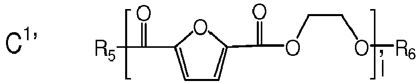 or the monomer component D¹ comprises the specific structure 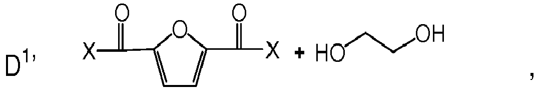 and the structure Y¹ of the cyclic polyester oligomer having furanic units is the specific structure 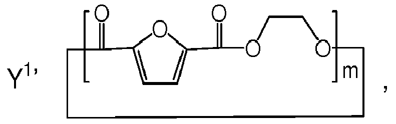 wherein
R₅= OH, OR, halogen, or O-CH₂CH₂-OH,
R = optionally substituted linear, branched or cyclic alkyl, phenyl, aryl or alkylaryl,
R₆= H or 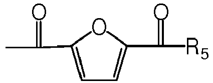 and X, I, and m are defined as indicated in the previous claim(s) on which this claim depends.

6. The process of any one of claims 1 to 4, wherein either the monomer component C¹ comprises the specific structure C¹" 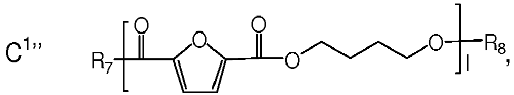 or the monomer component D¹ comprises the specific structure D¹" 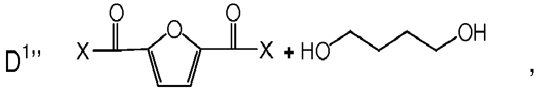 and the structure Y¹ of the cyclic polyester oligomer having furanic units is the specific structure Y¹" 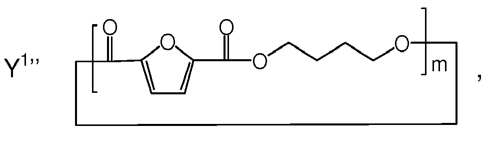 R₇= OH, OR, halogen, or O-CH₂CH₂ CH₂CH₂-OH,
R = optionally substituted linear, branched or cyclic alkyl, phenyl, aryl or alkylaryl,
R₈= H or 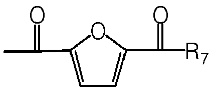 and X, I, and m are defined as indicated in the previous claim(s) on which this claim depends.

7. The process of any one of claims 1 to 6, wherein the optional organic base E is present and it is a monoamine compound or a compound having the structure 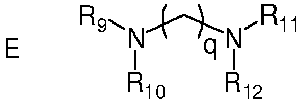 wherein each of the groups R₉ to R₁₂ are hydrogen, optionally-substituted alkyl, phenyl, aryl, or alkaryl, and wherein each of the groups R₉ to R₁₂ may optionally be bonded together by a single or double bond group as part of a cyclic substituent in a cyclic optional organic base E.

8. The process of any one of claims 1 to 7, wherein the optional organic base E is present and it is either:
(i) DABCO, having the structure: 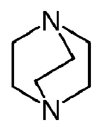 OR
(ii) DBU, having the structure: , 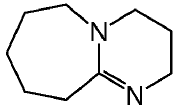 and wherein DABCO or DBU are optionally used together with an alkyl amine, more preferably triethylamine.

9. The process of any one of claims 1 to 8, wherein the optional catalyst is either absent or it is present and it is a metal alkoxide or metal carboxylate, preferably one of tin, zinc, titanium, or aluminium.

10. The process of any one of claims 1 to 9, wherein the optional organic base E is present in a stoichiometric ratio of from 0.5 to 6, preferably 1 to 4, more preferably 2 to 3 mol relative to 1 mol of all monomer component species used as a reactant in the process.

11. The process of anyone of claims 1 to 10, wherein the step (III) in which linear oligomeric polyester species having furanic units are separated and removed from the cyclic oligomeric composition comprises one or more additional separation sub-steps of passing a mobile phase of the cyclic oligomeric composition through a stationary phase, selective precipitation, distillation, extraction, crystallization or their combinations.

12. A process to produce a polyester polymer comprising (i) the process of any one of claims 1 to 11 to prepare a cyclic oligomer composition comprising a cyclic polyester oligomer having furanic units together with (ii) a subsequent polymerization step to produce a polyester polymer.

13. The process of claim 12, wherein the subsequent polymerization is carried out in the presence of a plasticizer.

14. The process of claim 13, wherein the plasticizer is one or more selected from the group consisting of a supercritical fluid and a polyether.

15. The process of claim 14, wherein the supercritical fluid is carbon dioxide or the polyether is a glyme, preferably tetraethylene glycol dimethyl ether.

16. The process of any one of claims 12 to 15, wherein the subsequent polymerization step is carried out in the presence of a catalyst, preferably wherein the catalyst is selected from a cyclic dibutyltin compound, Sb₂O₃, and SnOct₂, more preferably wherein the cyclic dibutyltin compound is 1,1,6,6-Tetra-n-butyl-1,6-distanna-2,5,7,10-tetraoxyacyclodecane.

17. A cyclic polyester oligomer composition obtainable, preferably obtained, by any one of claims 1 to 11, wherein the composition contains:
(i) a residual solvent in a concentration of less than 5, preferably 2, more preferably 1 wt %, and selected from the group consisting of an ionic liquid, an optionally-substituted napthalene, optionally-substituted aromatic compound, and their mixtures,
(ii) linear oligomeric polyester species having furanic units and present in a concentration of less than 5 %, preferably 3, most preferably 1 wt %,
and (iii) optionally a zeolite, in a concentration of less than of less than 5, preferably 2, more preferably 1 wt %, wherein the weight percentages are relative to the total weight of the cyclic polyester oligomer composition.

18. A polyester polymer composition obtainable, preferably obtained, by the process of any one of claims 12 to 16, wherein the composition contains:
(i) a plasticizer selected from the group consisting of an optionally-substituted phenyl ether, an ionic liquid, an optionally-substituted xylene, a polyether, and their mixtures,
(ii) a cyclic polyester oligomer having furanic units,
and (iii) EITHER:
(a) a PEF polymer comprising the structure 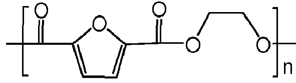 OR
(b) a PBF polymer comprising the structure 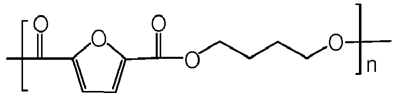 wherein n is an integer from 10 to 100,000, preferably 100 to 10,000.
